# EUROPEAN PATENT APPLICATION

(11) **EP 3 037 061 A1**
(43) Date of publication of application: **29.06.2016**
(21) Application number: 14199598.5
(22) Date of filing: 22.12.2014
(51) Int. Cl.: A61F 2/00

(54) **Resorbable medical mesh implant for repair or prevention of parastomal hernia**

(71) Applicant: Novus Scientific AB, 754 50 Uppsala (SE)
(72) Inventor: Mathisen, Torbjörn, 125 42 Älvsjö (SE)
(74) Representative: Brann AB

(57) **Abstract**

The invention relates to a degradable medical mesh implant for repair or prevention of a parastomal hernia in an individual, comprising an inner portion, which comprises a first mesh structure, and an outer portion, which surrounds the inner portion and comprises a second mesh structure. The degradable medical mesh implant is characterized in that the first mesh structure has a first value of a specific mechanical characteristic and that the second mesh structure has a second value of said specific mechanical characteristics, which second value of said specific mechanical characteristic is different from the first value of said specific mechanical characteristic.

## Description

### Field of the Invention

The present invention relates to a medical mesh device for the repair or prevention of parastomal hernia, and in particular to a resorbable medical mesh implant for the repair or prevention of parastomal hernia comprising at least two mesh portions with different mechanical characteristics, and even more particularly to a resorbable and preferably synthetic medical mesh implant for the repair or prevention of parastomal hernia comprising a central mesh portion characterized by having a low bending stiffness and a surrounding circumferential mesh portion characterized by having a high bending stiffness.

### Background of the Invention

Today, the creation of an ostomy in the abdominal wall of a human patient is a common medical procedure in both emergent and elective surgery. The ostomy may be intended to be permanent, but is also used to solve urgent situations, as protection of an intestinal anastomosis or preparatory to definite surgery with the aim of restoring bowel continuity at a later stage. However, the formation of an ostomy is often associated with complications, one of which is the development of a parastomal hernia. To prevent or at least reduce the risk of development of a parastomal hernia, the surgeon can choose to prophylactically implant a prosthetic mesh in a sublay position in the abdominal wall (i.e. in the preperitoneal plane created between the rectus muscle and the posterior rectus sheath); and especially with low-weight, large-pore meshes good clinical results have been reported. Medical meshes used for this purpose are medical standard meshes which are commercially available under trademarks such as Vypro™, which is a pliable and lightweight polypropylene and polyglactin multifilament mesh, and Ultrapro™, which is a lightweight polypropylene and poliglecaprone monofilament mesh. Although the implantation of such a lightweight mesh at the site of a stoma reduces the risk of development of a parastomal hernia, the commercially available meshes are typically designed to be applicable in a variety of medical situations, and can therefore be regarded as universal meshes. In other words, these commonly used meshes are not tailored and optimized for the specific medical situation prevailing at a stoma site when it comes to, for example, user-friendliness and tissue sensitivity.

There are also meshes specifically designed for the repair of a parastomal hernia once it has developed. Such an implantable mesh device is the Bard® CK™ Parastomal Hernia Patch, which comprises a monofilament polypropylene layer on the anterior muscle surface, a permanent ePTFE layer on the visceral surface, a central opening for accommodating a stoma, and a so-called memory recoil ring, which allows the patch to spring into place and lie flat against the abdominal wall. Although this device is specifically designed for the repair of a parastomal hernia it has several drawbacks. For example, the size of the central opening can only be moderately adjusted to accommodate the size of the stoma at hand, and several patches with different opening sizes must therefore be provided by the manufacturer and, more importantly, held readily available by the hospital. There is also a risk that the comparatively hard and rigid collar around the central opening in the patch causes excessive irritation and inflammatory reactions or even extrudes or erodes through the intestine accommodated therein. Similar risks are associated with the memory recoil ring which is adapted to lie embedded in the abdominal tissue surrounding the stoma.

Thus, there is still a need for an improved medical mesh for the repair or prevention of a parastomal hernia. The medical mesh should preferably have mechanical characteristics which are matched to the mechanical characteristics of the intestine accommodated within the mesh and also to the mechanical characteristics of the tissue surrounding the stoma. At the same time the improved medical mesh should be user-friendly and easy to position at the stoma site.

### Summary of the Invention

The above-mentioned objects are achieved by the present invention according to the independent claim. Preferred embodiments are set forth in the dependent claims.

A medical mesh device according to the present invention is intended to be implanted at the site of a stoma, which has been surgically created in the abdominal wall of an individual, in particular a human patient. More specifically, the medical mesh, which, for example, can have a circular, oval or rectangular shape, comprises a relatively small central portion, which is adapted to accommodate an intestine, such as the ileum or the colon, and a relatively larger surrounding portion, which, in a human patient, is adapted to be positioned in a sublay position, typically between the rectus abdominis muscle and the posterior rectus sheath, and is adapted to be anchored to the posterior rectus sheath. The central portion, which by the surgeon is cut open into a size which is matched to the size of the intestine to be brought there through, is characterized by being soft and pliable, not to cause irritation and excess inflammatory tissue response, whereas the larger surrounding portion is characterized by being stiffer and less pliable, to facilitate positioning and attachment to the tissue surrounding the stoma.

In one embodiment, a circular mesh implant is provided, which comprises a relatively small circular central portion, which is characterized by being relatively soft, compliant and elastic, and a surrounding larger portion, which is characterized by being rather stiff and inelastic. More specifically, the central portion has a comparatively low bending stiffness, whereas the surrounding portion has a comparatively high bending stiffness. The bending stiffness of the central portion is adapted to provide a soft and compliant contact surface to the intestine to be accommodated within the central portion once an opening has been cut out therein. The comparatively higher bending stiffness of the surrounding portion is adapted to facilitate easy positioning of the mesh implant behind the rectus abdominis muscle and provide for easy fastening, e.g. by suturing or stapling, to the abdominal wall tissue, e.g. to the posterior rectus sheath. In another embodiment, a rectangular mesh implant is provided, which comprises a relatively small oval central portion, in which a small cut has been made to make it easy for the surgeon to further cut open a central opening, the size of which is matched to the size of the intestine to be accommodated therein. As discussed above, the central portion has a comparatively low bending stiffness, whereas the surrounding portion has a comparatively high bending stiffness.

The different bending stiffnesses of a central portion and a surrounding portion, respectively, can be achieved by a dedicated heat-treatment process, in which a central portion is exposed to more heat than a surrounding portion. In yet another embodiment of the present invention, different bending stiffnesses are obtained by providing a mesh implant with a surrounding portion comprising two layers of mesh materials and a central portion comprising only one layer of mesh material. In still another embodiment, different bending stiffnesses are obtained by using a starting material which comprises different fibres made from different materials and dissolving or etching away certain types of these fibres in a central portion but not in a surrounding portion.

### Brief Description of the Drawings

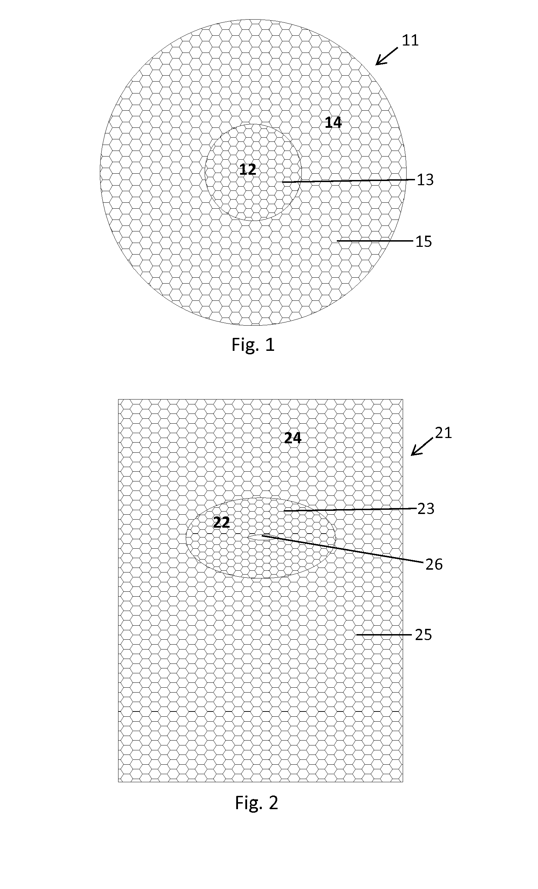

Fig. 1 shows a schematic picture of a first embodiment of a mesh implant according to the present invention, comprising a circular mesh structure having a circular central portion with a first value of a specific mechanical property and a surrounding circumferential portion with a second value of this specific mechanical property.
Fig. 2 shows a schematic picture of a second embodiment of a mesh implant according to the present invention, comprising a rectangular mesh structure having an oval, slightly off-centre portion with a first value of a specific mechanical property and a surrounding rectangular portion with a second value of this specific mechanical property.

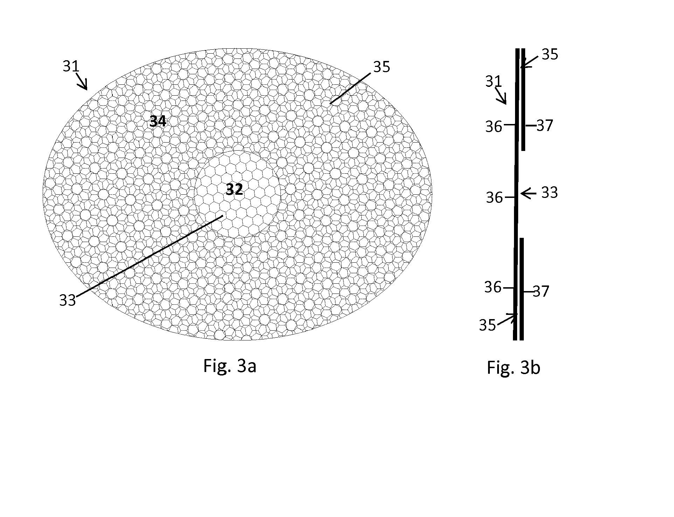

Fig. 3a shows a schematic front view of a third embodiment of a mesh implant according to the present invention, comprising an oval mesh structure having an oval central portion with a first value of a specific mechanical property and a surrounding oval portion with a second value of this specific mechanical property; and Fig. 3b shows a side view of this third embodiment, wherein it is illustrated that the surrounding oval portion comprises two layers of mesh materials and that the oval central portion comprises one layer of mesh material.

### Detailed Description of Preferred Embodiments

In general, a medical mesh implant according to the present invention comprises an inner portion, which, in turn, comprises a first mesh structure having a first value of a specific mechanical property or mechanical characteristic and an outer portion comprising a second mesh structure having a second value of the same mechanical property or mechanical characteristic. For all embodiments disclosed herein, it applies that instead of being restricted to only one mechanical property or one mechanical characteristic, a first mesh structure can also have a first set of values of a set of specific mechanical properties or mechanical characteristics, while a second mesh structure has a second set of values of the same set of specific mechanical properties or mechanical characteristics. The outer portion can virtually have any conceivable shape, but typically the outer portion is rectangular, quadratic, oval, or circular. The inner portion can also have any shape but has preferably an oval or circular shape. The inner portion can either, as in the embodiment described in conjunction with Fig. 1 below, assume a central position in a medical mesh implant, or can, as will be demonstrated in conjunction with Fig. 2 below, be positioned more or less off-centre.

An outer portion of a rectangular mesh can, for example, have a width of from 5 cm to 20 cm and a length of from 10 cm to 25 cm, while the inner portion can have a diameter of a few centimeters, for example a diameter of from 1 cm to 6 cm. A non-rectangular mesh, e.g. a circular or oval mesh, can have corresponding dimensions. Further, a medical mesh implant according to the invention is essentially a two-dimensional object with a thickness much lower than its width or length (or radius). The thickness of a mesh implant is typically only a few millimeters.

A first embodiment of medical mesh implant 11 according to the present invention is schematically illustrated in Fig. 1. The medical mesh implant 11 is designed and intended for the repair and prevention of a parastomal hernia in an individual, in particular a human patient, but can be used for other medical purposes as well. The mesh implant 11 comprises an inner portion 12, which has a circular shape and comprises a first mesh structure 13 having a first value of a specific mechanical property or characteristic, and an outer portion 14, which has a circular shape and comprises a second mesh structure 15 having a second value of the same specific mechanical property or characteristic. In this embodiment, the circular inner portion 12 is located in the centre of the mesh implant 11 and may therefore also be referred to as the central portion 12. Before use, a medical doctor or a surgeon or another qualified medical person, cuts out an opening in the central inner portion 12; and the size and shape of this opening is preferably matched to the size and shape of the intestine to be brought there through. When the intestine in question, e.g. an ileum or a colon, has been brought through the opening, the first mesh structure 13 will be in close contact with the outer surface of this intestine. In order not to cause an excessive inflammatory reaction and to avoid any risk that the first mesh structure 13 extrudes or erodes through the intestine due to intrinsic mechanical module mismatch between the first mesh structure 13 and the intestine, the first mesh structure 13 is characterized by being relatively soft, pliable, and mechanically compliant. The outer portion 14, which surrounds the inner portion 12 and which therefore may be referred to as the surrounding portion 14, is by the surgeon typically placed in a sublay position beneath the rectus abdominis muscle. To facilitate this positioning and to avoid wrinkles and buckles and to also facilitate easy fastening, e.g. by suturing or stapling, to some abdominal tissue such as the posterior rectus sheath, the second mesh structure 15 is characterized by being relatively stiff and rigid and less mechanically compliant than the first mesh structure 13.

A second embodiment of a medical mesh implant 21 according to the present invention is schematically illustrated in Fig. 2. Here the medical mesh implant 21 comprises an inner portion 22, which has an oval shape and comprises a first mesh structure 23 having a first value of a specific mechanical property or characteristic, and an outer portion 24, which has a rectangular shape and comprises a second mesh structure 25 having a second value of this specific mechanical property or characteristic. In this embodiment, the inner portion 22 is positioned slightly off-centre, towards one of the short sides of the rectangular mesh implant 21. Further, the inner portion 22 is provided with a through cut 26, which facilitates the further cutting out of an opening in the inner portion 22. For the same reasons as discussed above, the first mesh structure 23 is characterized by being relatively soft, pliable, and mechanically compliant, while the second mesh structure 25 is characterized by being relatively stiff and rigid and less mechanically compliant than the first mesh structure 23.

Different techniques can be utilized to produce a medical mesh implant according to the present invention, which is characterized by comprising at least two portions with different mechanical properties. A mesh implant according to the invention can be treated with heat in a process which in the art commonly is referred to as annealing, which typically is done by placing the mesh in a mould which is heated to a specific temperature. To produce portions with different mechanical characteristics, certain areas of the mesh material can be exposed to more (or less) heat, i.e. higher (or lower) temperature or longer (or shorter) dwell time in the mould. For example, a higher temperature and/or a longer dwell time can be provided for an outer portion than for an inner portion of a medical mesh implant, to thereby create a medical mesh implant comprising an inner portion characterized by being relatively soft, pliable, and mechanically compliant, and a surrounding or outer portion characterized by being relatively stiff, rigid, and less mechanically compliant than the inner portion.

Furthermore, from the above discussion about utilizing an annealing process to accomplish a medical mesh implant in accordance with the invention, it should be apparent that a mesh structure in an inner portion can be visually equal to a mesh structure in an outer portion, while the differences between these two portions manifest themselves through different intrinsic properties such as different morphologies. In other words, the difference between a first mesh structure in an inner portion of a mesh implant and a second mesh structure in an outer portion is established by differences in one or more mechanical properties or characteristics rather than by possible differences in visual appearances.

In Figs. 3a and 3b another way of accomplishing a medical mesh implant comprising two portions with different mechanical characteristics is illustrated. Here, a third embodiment of medical mesh implant 31 according the invention comprises an inner portion 32, which has an circular shape and comprises a first mesh structure 33 having a first value of a mechanical characteristic, and an outer portion 34, which has an oval shape and comprises a second mesh structure 35 having a second value of this mechanical characteristic. As explained above in conjunction with the first embodiment shown in Fig. 1 and the second embodiment shown in Fig. 2, the first mesh structure 33 is characterized by being relatively soft, pliable, and mechanically compliant, whereas the second mesh structure 35 is characterized by being relatively stiff, rigid, and less mechanically compliant than the first mesh structure 33.

In this third embodiment of the invention, the differences in mechanical characteristics have, however, not been achieved by different annealing cycles and/or temperatures for the first mesh structure 33 and the second mesh structure 35, respectively. Instead, as is most clearly illustrated in Fig. 3b, the second mesh structure 35 comprises two layers of mesh material, i.e. a first mesh layer 36 and a second mesh layer 37, whereas the first mesh structure 33 comprises only the first mesh layer 36. The second mesh layer 37 can be stiffer and more rigid than the first mesh layer 36, to provide an aggregate second mesh structure 35, which is characterized by being relatively stiff, rigid, and less mechanically compliant than the first mesh structure 33. It should however be noted that even if a second mesh layer is arranged, which is relatively soft and compliant in itself, the combination of a first mesh layer 36 and a second mesh layer 37 can result in an aggregate second mesh structure 35 which fulfils the requirement of a mesh implant according to the present invention by being relatively stiff, rigid, and less mechanically compliant than the first mesh structure 33. In a more general embodiment of a mesh implant comprising mesh layers (not shown in the drawings), also an inner portion can comprise more than one layer of mesh, e.g. two or three layers of mesh, and an outer layer can comprise these layers of mesh as well as at least one extra layer of mesh.

Another way of creating a medical mesh implant comprising two portions with different mechanical characteristics is to utilize a mesh already comprising two different fibres made from two different materials as a starting material. Two-component meshes are not commonly available in the market. However, one such two-component mesh product is TIGR^{®} Matrix Surgical Mesh, which is a synthetic, resorbable, two-component mesh manufactured and sold by the company Novus Scientific. This product and similar mesh products are described in U.S. Patent No. 8,016,841. Starting with a two-component mesh, wherein a first knit pattern comprises one or more fibres made from a first material or a first set of materials and a second knit pattern comprises one or more fibres made from a second material or a second set of materials, and wherein the first material or the first set of materials is different from the second material or the second set of materials, it is possible to dissolve or etch away the second material or the second set of materials by using a suitable solvent or etcher, which dissolves or etches away the second material or the second set of materials while leaving the first material or the first set of materials unaffected and thereby also leaving the first knit pattern intact. For example, TIGR^{®} Matrix Surgical Mesh is knitted from two different fibres arranged in two different knit patterns. The first fibre is a copolymer of lactide, and trimethylene carbonate, and the second fibre is a copolymer of glycolide, lactide, and trimethylene carbonate. By applying an etcher, such as, but not limited to, a phosphate buffer (e.g. 70 g dibasic potassium phosphate dissolved in 2000 ml water and adjusting the pH to 12 with 5 M sodium hydroxide solution), on a restricted area of a TIGR^{®} Matrix Surgical Mesh product, the second fibre and thereby the second knit pattern can be dissolved or etched away in one (inner) portion of this mesh product, leaving only the first fibre and thereby the first knit pattern intact in this limited and well-defined portion. More specifically, by applying a phosphate buffer, on an inner portion of a TIGR^{®} Matrix Surgical Mesh product, a medical mesh implant according to the present invention can be created. Other examples of buffer systems that can be used are carbonate buffers and amine buffers with a pH in the range from 8 to 14, but more preferably with a pH in the range from 11 to 13.

In the above specific case, wherein the first knit pattern comprises one or more fibres made from a first material or a first set of materials and a second knit pattern comprises one or more fibres made from a second material or a second set of materials, and wherein the first material or the first set of materials is different from the second material or the second set of materials, such that the second material or the second set of materials is dissolvable in an organic solvent, the fibres of the second knit pattern can be dissolved, thereby leaving only the first knit pattern, which is made from the fibres made from the first material or the first set of materials, within the section or portion of the mesh product that was left without masking. A mask for the mesh product is most easily made by a water-soluble polymer such as, but not limited to, polyethyleneglycol, polypropylene glycol, polyvinylalcohol or polyvinylidenepyrrolidone. These examples of protective masking polymers can easily be extracted from the mesh using neutral water after the dissolution of the second set of fibres has been completed.

In its general form the present invention relates to a medical mesh implant comprising an inner portion comprising a first mesh structure characterized by a first value of a specific mechanical property or characteristic, or a first set of values of a set of specific mechanical properties or characteristics, and an outer portion, which surrounds the inner portions and comprises a second mesh structure characterized by a second value of this specific mechanical property or characteristic, or a second set of values of the set of these specific mechanical properties or characteristics, wherein the first value differs from the second value, or the first set of values differs from the second set of values. Above, these mechanical characteristics have for the first mesh structure been stated in terms of being relatively soft, pliable, and mechanically compliant, and the mechanical characteristics for the second mesh structure has been stated as being relatively stiff, rigid, and less mechanically compliant than the first mesh structure. In other words, the mechanical characteristics of the first and second mesh structures have been expressed in terms that are intuitively easy to understand, but may be difficult to quantify experimentally. The mechanical characteristics or properties of a mesh structure according to the invention include, but are not limited to, extension, modulus of elasticity, mechanical compliance, bending stiffness, yield strength, tensile strength, or elongation. Although all of these properties do have well-defined meanings, some of them may be difficult to measure in an unambiguous way due to the inherent complexity of a mesh structure, and - as consequence - its nonlinear response when exposed to loads and strains. It is, however, believed that bending stiffness is a quantity that is relatively easy to measure, and the mesh structures disclosed and described herein are therefore characterized in terms of their bending stiffnesses. The bending stiffness of a mesh structure can be measured according to the standard ASTM D1388 using the cantilever test. Usually knitted meshes have different mechanics when measured in the direction of knitting (warp direction) or in the direction of the needle bed (course direction). To avoid doubt, herein, bending stiffness always refers to the highest bending stiffness as measured in any of these two directions, i.e. warp direction or course direction. Also, depending on the knitting pattern, the ease of bending will be different depending on which side of the knitting pattern that faces downwards (direction of the gravity). Therefore, using the cantilever test as described in the ASTM D1388 standard, both sides of the test sample shall be tested an equal number of times and the mean bending stiffness shall be calculated and hereinafter be denoted as the bending stiffness of the test sample.

When measured according the ASTM D1388 standard, a first mesh structure according to the present invention, i.e. the mesh structure of an inner portion of a medical mesh implant, is preferably characterized by having a bending stiffness less than 15 MPa (15x10⁶ Pascal), and more preferably less than 10 MPa (10x10⁶ Pascal), and a second mesh structure according to the present invention, i.e. the mesh structure of a surrounding outer portion of a medical mesh implant, is preferably characterized by having a bending stiffness higher than 15 MPa (15x10⁶ Pascal), and more preferably higher than 20 MPa (20x10⁶ Pascal).

Fibres to be used in a mesh implant according to the present invention are preferably made from degradable polymers, which degrade or resorb in the human body once the mesh has been implanted. Even more preferred are synthetic degradable polymers. Non-limiting examples of polymers suitable for the meshes presented herein are synthetic resorbable polymers made from the monomers glycolide, lactide, and all stereoisomers thereof; trimethylene carbonate, ε-caprolactone, dioxanone or dioxepanone, or various combinations thereof. Yet other non-limiting examples of synthetic resorbable polymers that can be utilized are various aliphatic polyurethanes, such as polyureaurethanes, polyesterurethanes and polycarbonateurethanes, and also materials such as polyphosphazenes or polyorthoesters. Further non-limiting examples are polymers belonging to the group often referred to as polyhydroxyalkanoates, and more specifically poly-beta-hydroxybutyrate and poly-gamma-hydroxybutyrate, either in their pure forms or as copolymers.

Although the present invention has been described with reference to specific embodiments, also shown in the appended drawings, it will be apparent to those skilled in the art that many variations and modifications can be done within the scope of the invention as described in the specification and defined with reference to the claims below.

## Claims

1. A degradable medical mesh implant (11; 21; 31) for repair or prevention of a parastomal hernia in an individual, comprising an inner portion (12; 22; 32), which comprises a first mesh structure (13; 23; 33), and an outer portion (14; 24; 34), which surrounds the inner portion and comprises a second mesh structure (15; 25; 35), **characterized in that** the first mesh structure has a first value of a specific mechanical characteristic and that the second mesh structure has a second value of said specific mechanical characteristic, which second value of said specific mechanical characteristic is different from the first value of said specific mechanical characteristic.

2. The degradable medical mesh implant according to claim 1, wherein said specific mechanical characteristic can be anyone of extension, modulus of elasticity, mechanical compliance, bending stiffness, yield strength, tensile strength, or elongation.

3. The degradable medical mesh implant according to claim 1 or claim 2, wherein the specific mechanical characteristic is bending stiffness, and wherein the bending stiffness of the first mesh structure (13; 23; 33) is less than 15 MPa and the bending stiffness of the second mesh structure (15; 25; 35) is higher than 15 MPa.

4. The degradable medical mesh implant according to claim 3, wherein the bending stiffness of the first mesh structure (13; 23; 33) is less than 10 MPa and the bending stiffness of the second mesh structure (15; 25; 35) is higher than 15 MPa.

5. The degradable medical mesh implant according to claim 3, wherein the bending stiffness of the first mesh structure (13; 23; 33) is less than 10 MPa and the bending stiffness of the second mesh structure (15; 25; 35) is higher than 20 MPa.

6. The degradable medical mesh implant according to any one of the preceding claims, wherein the difference between the first value and the second value has been accomplished in an annealing process, in which the outer portion has been exposed to a longer annealing time and/or a higher annealing temperature than the inner portion.

7. The degradable medical mesh implant according to any one of the preceding claims, wherein the difference between the first value and the second value has been accomplished by providing one layer of mesh in the inner portion and providing two layers of mesh in the outer portion.

8. The degradable medical mesh implant according to any one of the preceding claims, wherein the difference between the first value and the second value has been accomplished in an etching or dissolving process, wherein the inner portion and the outer portion comprise at least a first type of fiber made from a first type of polymer or polymers and a second type of fiber made from a second type of polymer or polymers, and wherein the first type of fiber is dissolved or etched away in the inner portion but not in the outer portion.

9. The degradable medical mesh implant according to any one of claim 1 to claim 8, wherein the first mesh structure (13; 23; 33) and the second mesh structure (15; 25; 35) are made from any one of the monomers glycolide, lactide, or any stereoisomer(s) thereof; trimethylene carbonate, ε-caprolactone, dioxanone or dioxepanone, or any combination thereof; aliphatic polyurethane(s), such as polyureaurethane(s), polyesterurethane(s) or polycarbonateurethane(s); polyphosphazene(s) or polyorthoester(s); or one or several polymers belonging to the group of polyhydroxyalkanoates, e.g. poly-beta-hydroxybutyrate and/or poly-gamma-hydroxybutyrate, either in their pure forms or as copolymers.
